# EUROPEAN PATENT APPLICATION

(11) **EP 1 932 823 A1**
(43) Date of publication of application: **18.06.2008**
(21) Application number: 06810542.8
(22) Date of filing: 26.09.2006
(51) Int. Cl.: C07C 51/12, C07C 53/08, C07B 61/00

(54) **PROCESS FOR PRODUCTION OF ACETIC ACID**

(30) Priority: 03.10.2005 JP 2005289584
(71) Applicant: Daicel Chemical Industries, Ltd., Kita-ku, Osaka-shi Osaka 530-0001 (JP)
(72) Inventor: KOJIMA, Hidetaka, Himeji-shi Hyogo 6700803 (JP)
(74) Representative: Grünecker, Kinkeldey, Stockmair & Schwanhäusser Anwaltssozietät
(86) International application number: PCT/JP2006/319017
(87) International publication number: WO 2007/040087

(57) **Abstract**

A process produces acetic acid by continuously carrying out a reaction of methanol with carbon monoxide in the presence of a Group VIII metal catalyst, an iodide salt, methyl iodide, and water in a reactor, continuously withdrawing a reaction mixture from the reactor, introducing the reaction mixture into an evaporation process at a pressure lower than that in the reaction to separate the reaction mixture into low-boiling components and high-boiling components containing the Group VIII metal and the iodide salt, and recycling the separated high-boiling components containing the Group VIII metal and the iodide salt to the reactor, in which the separated high-boiling components are brought into contact with hydrogen at temperatures of 80°C or higher for 6 seconds or longer before the high-boiling components reaching the reactor, which hydrogen is introduced in an amount of 0.1 time by mole or more that of the Group VIII metal. According to the process, industrially, acetic acid is efficiently produced with high productivity, because the activity of a catalyst in a reactor may be increased without increasing a hydrogen partial pressure in the reactor more than necessary, and a shift reaction may be suppressed to thereby reduce by-products.

## Description

### Technical Field

The present invention relates to processes for the production of acetic acid from methanol and carbon monoxide.

### Background Art

Acetic acid is one of basic chemicals and is important typically in the industries of petrochemistry, polymer chemistry, organic chemistry, and production of pharmaceutical and agricultural chemicals. Of various processes for the production of acetic acid, a process for the production of acetic acid from methanol and carbon monoxide is an industrially most effective process.

For improving this process, techniques of reducing the water content of a reaction mixture are disclosed (in Japanese Examined Patent Application Publication (JP-B) No. Hei 04-69136 and Japanese Examined Patent Application Publication (JP-B) No. Hei 07-23337). Specifically, they are techniques of reducing the water content in a reaction mixture to thereby produce acetic acid at higher productivity and to reduce by-products. These documents also teach that the stability of a rhodium catalyst decreases at a water content of 10 percent by weight or less, and disclose a technique of adding an alkali metal iodide, a quaternary ammonium salt, and/or a quaternary phosphonium salt, to avoid this problem effectively. In addition, they teach that the reaction rate significantly decreases at a water content of the reaction mixture of 10 percent by weight or less and disclose a technique of increasing the reaction rate by using 5 to 30 percent by weight of lithium iodide.

According to a regular industrial process for the production of acetic acid from methanol and carbon monoxide, methanol and carbon monoxide are continuously fed to a reaction mixture in a reactor to carry out a reaction; the reaction mixture is continuously withdrawn from the reactor and introduced into an evaporation tank (e.g. a flasher) under a pressure lower than that in the reactor to separate the reaction mixture into components which evaporate at the lower temperature (low-boiling components), and other components which do not (high-boiling components). The low-boiling components mainly contain methyl iodide as a promoter, methyl acetate derived from starting material methanol, water contained in the reaction mixture, and acetic acid as a product and as a reaction solvent. The high-boiling components contain, for example, unevaporated residual components to be contained in the low-boiling components, such as methyl iodide, methyl acetate, water, and acetic acid, as well as the catalyst rhodium complex and lithium iodide as a stabilizer for rhodium.

However, such an industrial continuous reaction, if carried out at a water content of 10 percent by weight or less, invites a gradually decreasing reaction rate. This is because rhodium of the main catalyst complex is converted from active monovalent in the reaction into inactive trivalent. When a reaction is carried out at a high water content, material carbon monoxide undergoes a shift reaction with water to form hydrogen and carbon dioxide. The formed hydrogen acts to convert trivalent rhodium into monovalent rhodium. Specifically, the activity of the catalyst can be maintained at a high production of hydrogen, because the inactive trivalent rhodium is rapidly converted into a monovalent rhodium. In contrast, when a reaction is carried out at a low water content, hydrogen production decreases, the trivalent rhodium is not so rapidly converted into a monovalent rhodium, and the catalytic activity and the reaction rate gradually decrease. In addition, the trivalent rhodium is converted into insoluble rhodium iodide.

To solve the problems due to the reduced water content of the reaction mixture, a technique of feeding hydrogen to the reaction system to thereby maintain the hydrogen partial pressure in the reaction system to a predetermined level or higher, is disclosed(in Japanese Examined Patent Application Publication (JP-B) No. Hei 08-5839). This technique maintains the rate of converting trivalent rhodium into monovalent rhodium at a certain level and thereby maintains the reaction activity. However, feeding hydrogen to the reactor to thereby maintain the hydrogen partial pressure to a high level cancels one of the advantages of reducing the water content of the reaction mixture, i.e., the reduction of by-products. Specifically, by reducing the water content of the reaction mixture, the shift reaction concerning carbon monoxide and water is suppressed to thereby reduce the hydrogen partial pressure; and this reduces by-products, such as propionic acid, formic acid, and hydrocarbons, formed as a result of a hydrogenation reaction. The procedure of feeding hydrogen to the reactor cancels this advantage of reducing by-products. In other words, when hydrogen is fed to the reactor to keep the hydrogen partial pressure to a specific level or higher to thereby maintain the activity of rhodium in a reaction carried out at a water content of 10 percent by weight or less, by-products such as formic acid, propionic acid, and hydrocarbons increase in proportion with an increasing hydrogen partial pressure in the reactor.

A process of carrying out evaporation to separate high-boiling components containing rhodium; treating the high-boiling components with hydrogen at least at a hydrogen partial pressure of 0.1 atmosphere or more and with carbon monoxide at a 0.1 atmosphere or more; and recycling the treated components to a reactor, is disclosed (in Japanese Patent No. 3213392). In Examples of this patent, a gaseous mixture of hydrogen and carbon monoxide at atmospheric pressure is introduced into a liquid corresponding to a catalyst circulating liquid, and a treatment is carried out at 140°C for 30 minutes. The processing time herein is, if employed in an industrial process, is such as to require a large vessel for treatment.

Patent Document 1: Japanese Examined Patent Application Publication (JP-B) No. Hei 04-69136
Patent Document 2: Japanese Examined Patent Application Publication (JP-B) No. Hei 07-23337
Patent Document 3: Japanese Examined Patent Application Publication (JP-B) No. Hei 08-5839
Patent Document 4: Japanese Patent No. 3213392

### Disclosure of Invention

### Problems to be Solved by the Invention

Accordingly, an object of the present invention is to provide a process of industrially efficiently producing acetic acid with high productivity, which process increases the activity of a catalyst in a reactor without increasing a hydrogen partial pressure in the reactor more than necessary, and suppresses a shift reaction to thereby reduce by-products.

### Means for Solving the Problems

After intensive investigations to achieve the above objects, the present inventor has found that, in a process of producing acetic acid from methanol and carbon monoxide using a catalyst of a Group VIII metal, in the Periodic Table of Elements, such as a rhodium compound, the Group VIII metal such as rhodium becomes trivalent and inactive in an evaporation tank (e.g. a flasher) in which the partial pressure of carbon monoxide is lower than that in a reactor, or on the way for a catalyst fluid containing the compound of Group VIII metal to be returned from the evaporation tank to the reactor. The present inventor has also found that the trivalent Group VIII metal contained in the catalyst fluid is to be reacted by contacting with a specific amount of hydrogen for a very short time on the way from the evaporation tank to the reactor so as to convert the trivalent Group VIII metal into a monovalent metal; the specific amount of hydrogen is consumed as a result of the reaction with the trivalent Group VIII metal before it reaches the reactor and is not so high as partial pressure in the reactor; and the contact time herein is so very short as to eliminate the need for a special vessel and to be effective even if a regular pipe line is used. Specifically, the present inventor has found that this configuration increases the activity of the catalyst in the reactor without increasing the hydrogen partial pressure in the reactor more than necessary, reduces the shift reaction, reduces by-products such as acetaldehyde, propionic acid, formic acid, and hydrocarbons, and reduces accumulation of unsaturated compounds such as crotonaldehyde. The present invention has been achieved based on these findings.

Specifically, the present invention provides a process for the production of acetic acid, the process comprising the steps of continuously carrying out a reaction of methanol with carbon monoxide in the presence of a catalyst of Group VIII metal of the Periodic Table of Elements, an iodide salt, methyl iodide, and water in a reactor, continuously withdrawing a reaction mixture from the reactor, introducing the reaction mixture into an evaporation process at a pressure lower than the pressure in the reaction to separate the reaction mixture into low-boiling components and high-boiling components containing the Group VIII metal and the iodide salt, and recycling the separated high-boiling components containing the Group VIII metal and the iodide salt to the reactor, in which the separated high-boiling components are brought into contact with hydrogen at temperatures of 80°C or higher for 6 seconds or longer in the step of recycling before the high-boiling components reaching the reactor, and the hydrogen is introduced in an amount of 0.1 time by mole or more that of the Group VIII metal.

The Group VIII metal catalysts include rhodium catalysts. The process for the production of acetic acid is highly advantageous when a reaction is carried out at a water content of a reaction mixture of 0.1 to 10 percent by weight. The concentration of the Group VIII metal catalyst in the reaction mixture is preferably about 300 to about 3000 ppm by weight in terms of metal. The iodide salt can be, for example, lithium iodide.

### Advantages

According to the present invention, the activity of the catalyst in the reactor can be increased without increasing the hydrogen partial pressure in the reactor more than necessary, because the catalyst fluid withdrawn from the evaporator is brought into contact with hydrogen before it is recycled to the reactor. Accordingly, there is no increase in hydrogen-induced by-products such as acetaldehyde, formic acid, propionic acid, hydrocarbons and increase in unsaturated compounds such as crotonaldehyde as a secondary by-product of acetaldehyde. The present invention further realizes a reduced water content in the reaction system without inactivating the catalyst, enables the catalyst to be reused (regenerated) even at a reduced water content of the reaction system, and thereby improves the productivity of acetic acid as a result of reduction of the water content in the reaction system. The reduction in water content further suppresses the shift reaction (CO + H₂O → CO₂ + H₂) to thereby reduce the by-products induced by hydrogen formed in the shift reaction. The process according to the present invention uses hydrogen in an amount of 0.1 time by mole or more with respect to the Group VIII metal in the catalyst fluid to thereby activate the catalyst in a very short time. Thus, the process does not need large-sized facilities, can thereby activate the catalyst and increase the productivity of acetic acid without high cost.

### Best Mode for Carrying Out the Invention

Acetic acid is produced according to the present invention by reacting methanol with carbon monoxide in the presence of a Group VIII metal catalyst, an iodide salt, methyl iodide, and water. Methanol and carbon monoxide as raw materials are continuously fed to a reactor, respectively. The reactor is a gas-liquid mixing chamber and can be any of a CSTR (Continuous Stirred-Tank Reactor) equipped with a stirrer, a mixed flow reactor of liquid circulation system without stirrer, and a bubble tower reactor. The reaction temperature is generally about 150°C to about 230°C, and preferably about 170°C to about 220°C. The reaction pressure is, in terms of total pressure, generally about 1.5 to about 5 MPa, and preferably about 2 to about 3.5 MPa.

The Group VIII metal catalyst and methyl iodide serve as a main catalyst and a promoter (co-catalyst), respectively. The iodide salt serves as a stabilizer and a promoter. Group VIII metals include iron, cobalt, nickel, ruthenium, rhodium, palladium, osmium, iridium, and platinum. Among them, platinum group elements such as ruthenium, rhodium, palladium, osmium, iridium, and platinum are preferred, of which rhodium is more preferred.

Such a Group VIII metal catalyst generally exists as a Group VIII metal complex in a reaction mixture. Accordingly, the Group VIII metal catalyst can be any Group VIII metal complex capable of dissolving in a reaction mixture under reaction conditions or any one capable of forming the complex of Group VIII metal. More specifically, the Group VIII metal complex is preferably a rhodium iodine complex or rhodium carbonyl complex such as RhI₃ or [Rh(CO)₂I₂]⁻ when a rhodium catalyst is taken as an example. The amount of the Group VIII metal catalyst is generally about 300 to about 3000 ppm by weight and, for example, about 300 to about 2000 ppm by weight in terms of concentration as metal in the reaction mixture. The amount is preferably about 500 to about 2000 ppm by weight for high productivity, because an excessively high concentration of the Group VIII metal may often invite precipitation of an iodide of the metal, such as rhodium iodide.

Methyl iodide is preferably used in a concentration in the reaction mixture of about 5 to about 20 percent by weight. Although the reaction is more accelerated with an increasing concentration of methyl iodide, economically most advantageous concentrations are preferably set from the viewpoints of size of facilities for the recovery and circulation into the reactor of methyl iodide and the quantity of energy used. The reaction mixture contains 0.1 to 30 percent by weight of methyl acetate as a result of the equilibrium between material methanol and product acetic acid.

The process according to the present invention controls the water content of the reaction mixture at, for example, about 0.1 to about 10 percent by weight, and preferably about 0.1 to about 5 percent by weight. With a decreasing water content, hydrogen formation as a result of the shift reaction decreases to thereby reduce by-products such as formic acid, propionic acid, and hydrocarbons, but the reaction rate decreases and the Group VIII metal catalyst becomes more unstable in many cases. To avoid these disadvantages, an iodide salt is used for accelerating the reaction and for stabilizing the Group VIII metal catalyst. The iodide salt can be any one that forms an iodide ion in the reaction mixture and includes, for example, iodide salts of alkali metals, such as LiI, NaI, KI, RbI, and CsI; iodide salts of alkaline earth metals, such as BeI₂, MgI₂, and CaI₂; and iodide salts of aluminum-group metals, such as BI₃ and AlI₃. In addition to the metal iodide salts, the iodide salt further includes iodide salts of organic substances. Examples thereof are quaternary phosphonium iodide salts including methyl iodide adducts or hydrogen iodide adducts of phosphines such as tributylphosphine and triphenylphosphine; and quaternary ammonium iodide salts including methyl iodide adducts or hydrogen iodide adducts of nitrogen-containing compounds such as tertiary amines, pyridines, imidazoles, and imides. Among them, iodide salts of alkali metals, such as LiI (lithium iodide), are preferred. The amount of the iodide salt is such that the iodide ion content in the reaction mixture is, for example, about 0.07 to about 2.5 mol/L, and preferably about 0.25 to about 1.5 mol/L and that the concentration of the iodide salt in the reaction mixture is about 3 to about 40 percent by weight, and preferably about 4.5 to about 30 percent by weight.

The reaction solvent is generally acetic acid as a product, but can be any solvent that does not adversely affect the reaction and separation/purification procedures.

The reaction mixture is continuously withdrawn from the reactor and is introduced, generally through valves and pipes, to an evaporation tank (e.g. a flasher) at a pressure lower than that in the reaction. The pressure in the evaporation tank is, for example, about 0.05 to about 0.3 MPaG (gauge pressure). Of the reaction mixture withdrawn from the reactor, low-boiling components including most of methyl iodide and methyl acetate, and part of water and acetic acid are separated in the evaporation process and are introduced into the steps of recovering low-boiling components and purifying product acetic acid. In the step of recovering low-boiling components, methyl iodide, methyl acetate, and water are separated and are circulated to the reactor generally using a pump, respectively. An unevaporated liquid component (circulating catalyst fluid) which has not been evaporated in the evaporation tank and contains the Group VIII metal and the iodide salt is also circulated to the reactor generally using a pump.

According to the present invention, the separated high-boiling components which have been separated in the evaporation process and contain the Group VIII metal and the iodide salt are brought into contact with hydrogen at temperatures of 80°C or higher for 6 seconds or longer before they reach the reactor in a zone from the evaporation tank to the reactor, which hydrogen is introduced in an amount of 0.1 time by mole or more that of the Group VIII metal. This procedure converts and regenerates the inactive trivalent Group VIII metal, such as rhodium, into an active monovalent Group VIII metal such as rhodium, which inactive trivalent metal is contained in the circulating catalyst fluid to be recycled to the reactor. The zone just mentioned above is preferably increased in pressure using a pump.

The concentration of the Group VIII metal catalyst (including inactive form) in the circulating catalyst fluid to be in contact with hydrogen is, for example, about 370 to about 5000 ppm by weight, and preferably about 600 to about 3300 ppm by weight in terms of metal.

The amount of hydrogen is about 0.1 time by mole or more, for example, about 0.1 to about 10 times by mole, preferably about 0.1 to about 5 times by mole, and more preferably about 0.5 to about 5 times by mole, with respect to the Group VIII metal such as rhodium. The process according to the present invention uses a specific amount or more of hydrogen with respect to the Group VIII metal, can thereby activate the catalyst rapidly, and can be used in small-sized devices or facilities. If the amount of hydrogen is less than 0.1 time by mole the amount of the Group VIII metal, the catalyst is not sufficiently activated, or the process requires large-sized facilities to ensure a satisfactory contact time to activate the catalyst. Hydrogen can be pure hydrogen gas or a gaseous mixture, such as an exhaust gas from the reactor, containing hydrogen and one or more other gases such as carbon monoxide, carbon dioxide, nitrogen, and/or methane. The hydrogen content of such a gaseous mixture, if used, is not specifically limited and is generally about 0.05 percent by volume or more, preferably about 1.0 percent by volume or more, and more preferably about 5.0 percent by volume or more. The pressure (total pressure) upon contact between the circulating catalyst fluid and hydrogen is, for example, about 0.05 MPa or more (e.g., about 0.05 to about 5 MPa), and preferably about 0.1 MPa or more (e.g., about 0.1 to about 5 MPa) and can be a pressure substantially equal to the reaction pressure. The hydrogen partial pressure of the hydrogen-containing gas for use in contact with the circulating catalyst fluid is generally about 0.0025 MPa or more, for example, about 0.0025 to about 5 MPa, and preferably about 0.01 MPa or more, for example, about 0.01 to about 5 MPa at an entrance of the contact zone. The temperature upon contact between the circulating catalyst fluid and hydrogen is 80°C or higher, for example, 80°C to 230°C, and preferably about 100°C to about 200°C. A contact at temperatures lower than 80°C fails to sufficiently activate the catalyst or requires large-sized facilities to ensure a sufficient contact time. The contact between the circulating catalyst fluid and hydrogen is carried out for 6 seconds or longer, for example, about 6 to about 600 seconds, and preferably about 30 to about 300 seconds. If the contact time is shorter than 6 seconds, the catalyst is not sufficiently activated. In this connection, an excessively long contact time requires a large-sized vessel for contact, and thereby the contact time is preferably 600 seconds or shorter.

The zone where the circulating catalyst fluid is brought into contact with hydrogen can comprise a regular gas-liquid mixing unit such as a jacketed pipe, or a static mixing device such as a static mixer. The circulating catalyst fluid after contact with hydrogen may be fed to the reactor without any treatment or may be subjected to gas-liquid separation, from which a liquid alone is separated, before being fed to the reactor.

### Examples

The present invention will be illustrated in further detail with reference to several Examples below, which by no means limit the scope of the present invention. The symbol "G" in the unit of pressure means a gauge pressure. The percentage (%) of a monovalent rhodium in rhodium complexes was determined by infrared absorption spectrometry. More specifically, rhodium complexes were precipitated using an aqueous tetraphenylphosphonium chloride solution, precipitates were separated and dried, and the infrared absorption spectrum was determined using a Fourier transform infrared spectrometer (FT-IR). The spectrum shows a peak derived from a complex containing monovalent rhodium at around 1970 cm⁻¹, a peak derived from a complex containing trivalent rhodium at around 2080 cm⁻¹, and peaks derived from the complexes containing monovalent and trivalent rhodium at around 2040 cm⁻¹ to 2030 cm⁻¹. Thus, the percentage of the monovalent rhodium was calculated based on the peak height (intensity) or peak area.

### Example 1

To a 28.8-ml reactor equipped with a heating jacket was continuously introduced a catalyst composition containing a rhodium complex having trivalency alone ([Rh(CO)₂I₄]⁻) as the catalyst in a concentration of 700 ppm by weight in terms of rhodium, and containing 86 percent by weight of acetic acid as a solvent, 2 percent by weight of water, and 12 percent by weight of lithium iodide; a 1:3 gaseous mixture of hydrogen and carbon monoxide was fed in such an amount that the ratio of hydrogen to rhodium was 2.3 times by mole; and the catalyst composition and the gaseous mixture were brought into contact with each other at temperatures shown in Table 1 at a pressure of 2.8 MPaG for 53 seconds. The percentage (%) of a monovalent rhodium as determined by infrared absorption spectrometry is shown in

[Table 1]

**Table 1**

| | | | | | |
|---|---|---|---|---|---|
| Contact temperature (°C) | 100 | 115 | 125 | 135 | 140 |
| Monovalent rhodium (%) | 35 | 61 | 72 | 78 | 82 |

### Example 2

To a 28.8-ml reactor equipped with a heating jacket was continuously introduced a catalyst composition containing a rhodium complex having trivalency alone as the catalyst in a concentration of 700 ppm by weight in terms of rhodium, and containing 86 percent by weight of acetic acid as a solvent, 2 percent by weight of water, and 12 percent by weight of lithium iodide; a 1:3 gaseous mixture of hydrogen and carbon monoxide was fed in such an amount that the ratio of hydrogen to rhodium was 1.1 times by mole; and the catalyst composition and the gaseous mixture were brought into contact with each other at a temperature of 125°C and a pressure of 2.8 MPaG for 53 seconds. The percentage (%) of a monovalent rhodium was determined by infrared absorption spectrometry to find to be 72%.

### Example 3

To a 1-liter reactor were continuously fed methanol (0.21 kg/h) as a reaction material, carbon monoxide, a catalyst fluid containing a rhodium catalyst and lithium iodide, and low-boiling components comprising methyl iodide (0.28 kg/h), methyl acetate (0.095 kg/h), and water (0.008 kg/h); a reaction was carried out at a reaction temperature of 196°C, a reaction pressure of 3.0 MPaG, and a hydrogen partial pressure of 29 kPa; a reaction mixture containing 0.45 percent by weight of water, 4.7 percent by weight of methyl acetate, 14.5 percent by weight of methyl iodide, 930 ppm by weight of the rhodium catalyst in terms of rhodium, and 11.7 percent by weight of lithium iodide was introduced to a flasher at a flow rate of 2.07 kg/h to thereby evaporate low-boiling components and formed acetic acid; and an unevaporated catalyst fluid containing 1480 ppm by weight of the rhodium catalyst in terms of rhodium was pressurized using a pump and was circulated to the reactor at a flow rate of 1.30 kg/h. In this procedure, the catalyst fluid was brought into contact with a 1:3 gaseous mixture of hydrogen and carbon monoxide (6.7 Nl/h) at a temperature of 135°C and a pressure of 3.0 MPaG for 53 seconds in a jacketed pipe between the flasher and the reactor. The ratio of hydrogen to rhodium was 4.0 times by mole.
Acetic acid, acetaldehyde, carbon dioxide formed as a result of shift reaction, and methane were produced at rates of 22.1 mol/L/h, 5.5 mmol/L/h, 10.8 mmol/L/h, and 39 mmol/L/h, respectively. The percentage of monovalent rhodium at the outlet of the reactor was 40%, as determined by infrared absorption spectrometry.

### Comparative Example 1

To a 1-L reactor were continuously fed methanol (0.20 kg/h) as a reaction material, carbon monoxide, a catalyst fluid containing a rhodium catalyst and lithium iodide, and low-boiling components comprising methyl iodide (0.26 kg/h), methyl acetate (0.096 kg/h), and water (0.007 kg/h); a reaction was carried out at a reaction temperature of 195°C, a reaction pressure of 3.0 MPaG, and a hydrogen partial pressure of 30 kPa; a reaction mixture containing 0.59 percent by weight of water, 5.2 percent by weight of methyl acetate, 13.6 percent by weight of methyl iodide, 780 ppm by weight of the rhodium catalyst in terms of rhodium, and 11.3 percent by weight of lithium iodide was introduced to a flasher at a flow rate of 2.04 kg/h to thereby evaporate low-boiling components and formed acetic acid; and an unevaporated catalyst fluid containing 1225 ppm by weight of the rhodium catalyst in terms of rhodium was pressurized using a pump and was circulated to the reactor at a flow rate of 1.30 kg/h.
Acetic acid, acetaldehyde, carbon dioxide formed as a result of shift reaction, and methane were produced at rates of 21.4 mol/L/h, 8.5 mmol/L/h, 9.9 mmol/L/h, and 43 mmol/L/h, respectively. The percentage of monovalent rhodium at the outlet of the reactor was 15%, as determined by infrared absorption spectrometry.

### Example 4

A methanol solution as a reaction material was prepared by bringing methanol into countercurrent contact with offgases, which were discharged from a 1-L reactor and a distillation column, at 5°C using an Ordershow column having an inner diameter of 40 mm and including ten trays, to thereby absorb methyl iodide, acetaldehyde, and other components in the offgases. To the reactor were continuously fed 91 Nl/h of carbon monoxide, the after-mentioned circulating catalyst fluid, a circulating fluid from the distillation column, and the methanol solution at a flow rate of 0.11 kg/h; a reaction was carried out at a reaction temperature of 186.5°C, a reaction pressure of 2.7 MPaG, and a hydrogen partial pressure of 28 kPa; a reaction mixture containing 1.8 percent by weight of water, 5.5 percent by weight of methyl acetate, 12.4 percent by weight of methyl iodide, 600 ppm by weight of the rhodium catalyst in terms of rhodium, and 9.8 percent by weight of lithium iodide was introduced to a flasher at a flow rate of 1.96 kg/h to thereby evaporate low-boiling components and formed acetic acid; and an unevaporated catalyst fluid having containing 820 ppm by weight of the rhodium catalyst in terms of rhodium was pressurized using a pump and was circulated to the reactor at a flow rate of 1.44 kg/h. In this procedure, the catalyst fluid was brought into contact with a 1:1 gaseous mixture of hydrogen and carbon monoxide (0.95 Nl/h) at a temperature of 90°C and a pressure of 2.7 MPaG for 53 seconds in a jacketed pipe between the flasher and the reactor. The ratio of hydrogen to rhodium was 1.8 times by mole. Components evaporated in the flasher were separated into formed acetic acid and low-boiling components such as methyl iodide, methyl acetate, and water using a distillation column, and the low-boiling components including acetaldehyde and butyraldehyde were circulated to the reactor at a flow rate of 0.33 kg/h.
Acetic acid, carbon dioxide formed as a result of shift reaction, and methane were produced at rates of 12.2 mol/L/h, 13.7 mmol/L/h, and 8.5 mmol/L/h, respectively. Butyraldehyde and crotonaldehyde were accumulated in the reaction mixture in concentrations of 48 ppm by weight and 2.5 ppm by weight, respectively. The percentage of monovalent rhodium at the outlet of the reactor was 45%, as determined by infrared absorption spectrometry.

### Example 5

To a 1-L reactor were fed 97 Nl/h of carbon monoxide, the after-mentioned circulating catalyst fluid, a circulating fluid from a distillation column, and a methanol solution as a reaction material at a flow rate of 0.11 kg/h, which methanol solution had been prepared by allowing methanol to absorb methyl iodide, acetaldehyde and other components in the offgases from the reactor and the distillation column by the procedure of Example 4; a reaction was carried out at a reaction temperature of 184.2°C, a reaction pressure of 2.7 MPaG, and a hydrogen partial pressure of 34 kPa; a reaction mixture containing 1.9 percent by weight of water, 5.7 percent by weight of methyl acetate, 12.0 percent by weight of methyl iodide, 600 ppm by weight of the rhodium catalyst in terms of rhodium, and 9.7 percent by weight of lithium iodide was introduced to a flasher at a flow rate of 2.05 kg/h to thereby evaporate low-boiling components and formed acetic acid; and an unevaporated catalyst fluid containing 840 ppm by weight of the rhodium catalyst in terms of rhodium was pressurized using a pump and was circulated to the reactor at a flow rate of 1.45 kg/h. In this procedure, the catalyst fluid was brought into contact with a 1:1 gaseous mixture of hydrogen and carbon monoxide (1.16 Nl/h) at a temperature of 135°C and a pressure of 2.7 MPaG for 53 seconds in a jacketed pipe between the flasher and the reactor. The ratio of hydrogen to rhodium was 2.2 times by mole. Components evaporated in the flasher were separated into formed acetic acid and low-boiling components such as methyl iodide, methyl acetate, and water using a distillation column, and the separated low-boiling components including acetaldehyde and butyraldehyde were circulated to the reactor at a flow rate of 0.34 kg/h.
Acetic acid, carbon dioxide formed as a result of shift reaction, and methane were produced at rates of 12.3 mol/L/h, 11.0 mmol/L/h, and 13.5 mmol/L/h, respectively. Butyraldehyde and crotonaldehyde were accumulated in the reaction mixture in concentrations of 37 ppm by weight and 2.0 ppm by weight, respectively. The percentage of monovalent rhodium at the outlet of the reactor was 63%, as determined by infrared absorption spectrometry. Industrial Applicability

According to the present invention, industrially, acetic acid is efficiently produced with high productivity, because the activity of a catalyst in a reactor may be increased without increasing a hydrogen partial pressure in the reactor more than necessary, and a shift reaction may be suppressed to thereby reduce by-products.

## Claims

1. A process for the production of acetic acid, the process comprising the steps of:
continuously carrying out a reaction of methanol with carbon monoxide in the presence of a catalyst of Group VIII metal of the Periodic Table of Elements, an iodide salt, methyl iodide, and water in a reactor;
continuously withdrawing a reaction mixture from the reactor;
introducing the reaction mixture into an evaporation process at a pressure lower than the pressure in the reaction to separate the reaction mixture into low-boiling components and high-boiling components containing the Group VIII metal and the iodide salt; and
recycling the separated high-boiling components containing the Group VIII metal and the iodide salt to the reactor,
wherein the separated high-boiling components are brought into contact with hydrogen at temperatures of 80°C or higher for 6 seconds or longer in the step of recycling before the high-boiling components reaching the reactor, and wherein the hydrogen is introduced in an amount of 0.1 time by mole or more that of the Group VIII metal.

2. The process of claim 1, wherein the Group VIII metal catalyst comprises a rhodium catalyst.

3. The process of one of claims 1 and 2, wherein the reaction is carried out at a water content of the reaction mixture of 0.1 to 10 percent by weight.

4. The process of any one of claims 1 to 3, wherein the concentration of the Group VIII metal catalyst in the reaction mixture is 300 to 3000 ppm by weight in terms of metal.

5. The process of any one of claims 1 to 4, wherein the iodide salt comprises lithium iodide.
